# EUROPEAN PATENT APPLICATION

(11) **EP 2 659 875 A1**
(43) Date of publication of application: **06.11.2013**
(21) Application number: 11852574.0
(22) Date of filing: 09.11.2011
(51) Int. Cl.: A61K 8/19, A61Q 17/04

(54) **COSMETIC MATERIAL AND METHOD FOR PREPARING SAME**

(30) Priority: 27.12.2010 JP 2010289187
(71) Applicant: IHI Corporation, Tokyo 135-8710 (JP)
(72) Inventor: EGUCHI, Haruki, Tokyo 135-8710 (JP); FUCHIGAMI, Kenji, Tokyo 135-8710 (JP)
(74) Representative: MERH-IP Matias Erny Reichl Hoffmann
(86) International application number: PCT/JP2011/075855
(87) International publication number: WO 2012/090593

(57) **Abstract**

A cosmetic material that exhibits sufficient shielding effects against UV-A and UV-B, does not color a resultant cosmetic product even when blended in cosmetic materials, and will not result in a non-powdery finish when applied to the skin, and a method for producing such a cosmetic material are provided. It is a cosmetic material with at least part of InTaO₄ substituted with at least one element of Sc, Ti, V, Cr, Mn, Co, Cu, Ga, Ge, As, Y, Zr, Nb, Mo, Tc, Ru, Rh, Pd, Ag, Cd, Sn, Sb, Hf, W, Re, Os, Ir, Pt, Au, and Hg.

## Description

### [Technical Field]

The present invention relates to a cosmetic material having ultraviolet absorbing property and a method for producing the cosmetic material.

### [Background Art]

It has been conventionally known that ultraviolet radiation causes various changes to the skin. Dermatologically, working wavelengths of the ultraviolet radiation are classified into long-wavelength ultraviolet radiation of 400 nm to 320nm, mid-wavelength ultraviolet radiation of 320 nm to 290nm, and short wavelength ultraviolet radiation of 290 nm or lower and these groups of radiation are called UV-A, UV-B, and UV-C, respectively.

Normally, most of the ultraviolet radiation to which humans are exposed is sunlight; and of such ultraviolet radiation, the ultraviolet radiation that reaches the ground is the UV-A and UV-B, while the UV-C hardly reaches the ground because the UV-C is absorbed in an ozone layer. If the skin is exposed to a certain light quantity or more of the UV-A and UV-B of the ultraviolet radiation which reaches the ground, that causes erythema or blisters, also promotes the formation of melanin, and causes changes such as occurrence of pigmentation. Therefore, it is very important to protect the skin against the UV-A and UV-B in terms of the prevention of accelerated skin senescence and the prevention of occurrence of blemishes and freckles. In view of the above, various UV-A and UV-B absorbers have been developed.

Dibenzoyl-methane derivatives are known as existing UV-A absorbers. (For example, see Patent Document 1).

Furthermore, PABA derivatives, cinnamic acid derivatives, salicylic acid derivatives, camphor derivatives, urocanic acid derivatives, benzophenone derivatives, and heterocyle derivatives are known as UV-B absorbers. (For example, see Patent Document 2).

These UV-A and UV-B absorbers are blended with skin drugs for external treatment such as cosmetic materials and quasi-pharmaceutical products and then used.

On the other hand, fine-particle titanium oxide is blended with, for examples, cosmetic materials intended for UV protection by utilizing ultraviolet radiation shielding power of the fine-particle titanium oxide; however, its protection effect is weaker than that of organic ultraviolet radiation absorbers used for the same purpose and a large blending quantity is required to have a high ultraviolet radiation protection effect. Accordingly, if an ultraviolet radiation protection cosmetic material in which the fine-particle titanium oxide conventionally sold in the market is blended is applied to the skin, there is fear, for example, that unnatural paleness will occur. Also, the conventional fine-particle titanium oxide shields the wavelength of the UV-B area (320 nm to 290nm), but does not shield the UV-A area (400 nm to 320nm) sufficiently.

So, for example, iron-containing ultrafine-particle rutile-type titanium oxide and iron-containing titanium dioxide, and so on are suggested as titanium oxides which have superior ultraviolet radiation shielding effects and do not result in unnatural whiteness. (For example, see Patent Document 3 and 4).

Moreover, titanium oxide - ceric oxide composite sol is also suggested as a combination of titanium oxide and ceric oxide. (For example, see Patent Document 5).

Furthermore, a combination of a lower-level titanium oxide pigment, titanium oxide, and cerium oxide is also suggested. (For example, see Patent Document 6).

Furthermore, an ultraviolet radiation absorber made of a silicon cluster or a germanium cluster is also suggested. (For example, see Patent Document 7).

### [Citation List]

### [Patent Document]

[Patent Document 1] Japanese Patent Application Laid-Open (Kokai) Publication No. 05-247063
[Patent Document 2] Japanese Patent Application Laid-Open (Kokai) Publication No. 2003-212711
[Patent Document 3] Japanese Patent Application Laid-Open (Kokai) Publication No. 05-330825
[Patent Document 4] Japanese Patent Application Laid-Open (Kokai) Publication No. 07-69636
[Patent Document 5] Japanese Examined Patent Publication (Kokoku) No. 06-650
[Patent Document 6] Japanese Patent Application Laid-Open (Kokai) Publication No. 60-245671
[Patent Document 7] Japanese Patent Application Laid-Open (Kokai) Publication No. 2005-314408

### [Summary of Invention]

### [Problems to be Solved by the Invention]

However, iron-containing titanium oxide is superior in prevention of a white-masked look caused by the titanium oxide and in the UV-A area shielding property, but its red color because of the iron oxide is strong and becomes orange when blended with cosmetic materials and its color tone is closer to a foundation than to a general milky lotion. So, if the iron-containing titanium oxide is used as a makeup base, there is fear that, for example, it will affect the color tone of the foundation or might result in a somber makeup.

Moreover, since the form of titanium oxide - ceric oxide composite sol is sol, there are limitations on blending with cosmetic materials and there is still room for improvement in terms of durability.

Furthermore, regarding combinations of titanium oxide and cerium oxide, lower-level titanium oxide is a black pigment, an ultraviolet radiation absorber made of a silicon cluster or germanium cluster is not actually used for cosmetic materials, which are not intended for coloring, because the silicon cluster and the germanium cluster are black pigments. Also, these black pigments do not sufficiently absorb light of the UV-A area (400 nm to 320nm) according to their light absorption spectra.

The present invention was devised in light of the circumstances described above and it is an object to provide: a cosmetic material that has sufficient shielding effects against the UV-A and the UV-B, does not color a resultant cosmetic product even when blended in cosmetic materials, and will not result in a non-powdery finish when applied to the skin; and a method for producing such a cosmetic material.

### [Means for Solving the Problems]

In order to achieve the above-described object, the present invention provides a cosmetic material with at least part of InTaO₄ (indium tantalum oxide) substituted with at least one element of Sc (scandium), Ti (titanium), V (vanadium), Cr (chromium), Mn (manganese), Co (cobalt), Cu (copper), Ga (gallium), Ge (germanium), As (arsenic), Y (yttrium), Zr (zirconium), Nb (niobium), Mo (molybdenum), Tc (technetium), Ru (rubidium), Rh (rhodium), Pd (palladium), Ag (silver), Cd (cadmium), Sn (tin), Sb (antimony), Hf (hafnium), W (tungsten), Re (rhenium), Os (osmium), Ir (iridium), Pt (platinum), Au (gold), and Hg (mercury). The cosmetic material having this structure can absorb the UV-A and the UV-B effectively.

Moreover, with the cosmetic material according to the present invention, at least part of In, Ta, or a combination thereof of InTaO₄ can be substituted with the abovementioned element(s). Alternatively, with the cosmetic material according to the present invention, part of In and Ta of InTaO₄ can be substituted with the abovementioned elements, respectively.

Furthermore, the present invention provides a cosmetic material with a material generated by substituting at least part of InTaO₄ with at least one element of Sc, Ti, V, Cr, Mn, Fe (iron), Co, Ni (nickel), Cu, Zn (zinc), Ga, Ge, As, Y, Zr, Nb, Mo, Tc, Ru, Rh, Pd, Ag, Cd, Sn, Sb, Hf, W, Re, Os, Ir, Pt, Au, and Hg, being in an oxygen deficiency state as a result of reduction processing. The cosmetic material having this structure can absorb the UV-A and the UV-B effectively.

Moreover, with the cosmetic material having the above-described structure, at least part of In, Ta, or a combination thereof of InTaO₄ can be substituted with the abovementioned element(s). Alternatively, with the cosmetic material according to the present invention, part of In and Ta of InTaO₄ can be substituted with the abovementioned elements, respectively.

Furthermore, the present invention provides a cosmetic material with InTaO₄ in an oxygen deficiency state as a result of reduction processing. The cosmetic material having this structure can absorb the UV-A and the UV-B effectively.

Furthermore, the present invention provides a cosmetic material with part of In or Ta of InTaO₄ or part of In and Ta of InTaO₄ is in a defect state. The cosmetic material having this structure can absorb the UV-A and the UV-B effectively.

Moreover, the present invention provides a cosmetic material production method including a substitution step of substituting at least part of InTaO₄ with at least one element of Sc, Ti, V, Cr, Mn, Co, Cu, Ga, Ge, As, Y, Zr, Nb, Mo, Tc, Ru, Rh, Pd, Ag, Cd, Sn, Sb, Hf, W, Re, Os, Ir, Pt, Au, and Hg. The cosmetic material capable of absorbing the UV-A and the UV-B effectively can be produced by this production method.

In the substitution step, a step of substituting at least part of In, Ta, or a combination thereof of InTaO₄ with the aforementioned element or a step of substituting at least part of In, Ta, or a combination thereof of InTaO₄ with the element can be performed.

Furthermore, the present invention provides a cosmetic material production method including a substitution step of substituting at least part of InTaO₄ with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg and an oxygen deficiency step of executing reduction processing to make the resultant material enter an oxygen deficiency state after the substitution step. The cosmetic material capable of absorbing the UV-A and the UV-B effectively can be produced by this production method.

Then, in the substitution step of this production method, a step of substituting at least part of In, Ta, or a combination thereof of InTaO₄ with the aforementioned element or a step of substituting at least part of In, Ta, or a combination thereof of InTaO₄ with the element can be performed.

Furthermore, the present invention provides a cosmetic material production method including an oxygen deficiency step of executing reduction processing on InTaO₄ to make the resultant material enter an oxygen deficiency state. The cosmetic material capable of absorbing the UV-A and the UV-B effectively can be produced by this production method.

### [Advantageous Effects of Invention]

The cosmetic material according to the present invention can absorb the UV-A and the UV-B effectively, so that it can exhibit sufficient shielding effects against the UV-A and the UV-B. Also, it is possible to provide the cosmetic material that does not color a resultant cosmetic product even when blended in cosmetic materials, and will not result in a non-powdery finish when applied to the skin.

The cosmetic material capable of exhibiting sufficient shielding effects against the UV-A and UV-B can be produced by the production method according to the present invention. Moreover, the cosmetic material that does not color the resultant cosmetic product even when blended in cosmetic materials, and will not result in a non-powdery finish when applied to the skin can be produced.

### [Brief Description of Drawings]

[Fig. 1] Fig. 1 illustrates the relationship between doping elements, which are derived based on a first principle calculation according to an embodiment of the present invention, and their bond energy.
[Fig. 2] Fig. 2 illustrates the relationship between electron energy and electron density when part of InTaO₄ is substituted with a doping element in the embodiment of the present invention.
[Fig. 3] Fig. 3 illustrates the relationship between the electron energy and the electron density when part of InTaO₄ is substituted with a doping element in the embodiment of the present invention.
[Fig. 4] Fig. 4 illustrates the relationship between the electron energy and the electron density when part of InTaO₄ is substituted with a doping element in the embodiment of the present invention.
[Fig. 5] Fig. 5 illustrates the relationship between the electron energy and the electron density when part of InTaO₄ is substituted with a doping element in the embodiment of the present invention.
[Fig. 6] Fig. 6 illustrates absorption spectra of cosmetic materials (samples) produced in the embodiment of the present invention by a diffuse reflectance spectroscopy method.
[Fig. 7] Fig. 7 illustrates the relationship between the electron energy and the electron density of InTaO₄ (no dope) and the relationship between the electron energy and the electron density when reduction processing is executed on InTaO₄, thereby making it enter an oxygen deficiency state, respectively, in the embodiment of the present invention.
[Fig. 8] Fig. 8 illustrates the relationship between the electron energy and the electron density of InTaO₄ (no dope), the relationship between the electron energy and the electron density when part of InTaO₄ is substituted with Ga as a doping element, and the relationship between the electron energy and the electron density when part of InTaO₄ is substituted with Ga as the doping element and the reduction processing is further executed on the resultant material, thereby making it enter the oxygen deficiency state, respectively.
[Fig. 9] Fig. 9 illustrates, in an enlarged manner, simulation results of an example where Ni and Ga are used as doping elements are representative examples of the elements indicated on the horizontal axis in Fig. 1.
[Fig. 10] Fig. 10 is a flowchart illustrating steps of producing the cosmetic material according to the present invention by a sol-gel method.

### [Description of Embodiments]

Next, a cosmetic material according to an embodiment of the present invention and a method for producing such a cosmetic material will be explained. Incidentally, in this embodiment, to introduce an element(s) to InTaO₄ and substitute part of InTaO₄ with the element is called "dope" and an element(s) which is used to substitute at least part of InTaO₄ may be sometimes called the "doping element(s)."

### <Cosmetic Material>

A cosmetic material according to the present invention is characterized in that it is a semiconductor cosmetic material with at least part of InTaO₄ substituted with at least one element (doping element) of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg. This cosmetic material may be one with only part of In of InTaO₄ substituted with the doping element or one with only part of Ta of InTaO₄ substituted with the doping element. Furthermore, the cosmetic material may be one with part of both In and Ta substituted with the doping elements. Furthermore, there may be one type of doping element or multiple types of doping elements to substitute part of InTaO₄.

Moreover, the cosmetic material according to the present invention is characterized in that reduction processing is executed on a material generated by substituting at least part of InTaO₄ with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg and the obtained material is thereby made to enter the oxygen deficiency state. This cosmetic material may be one with only part of In of InTaO₄ substituted with the doping element or one with only part of Ta of InTaO₄ substituted with the doping element. Furthermore, the cosmetic material may be one with part of both In and Ta substituted with the doping elements. Furthermore, there may be one type of doping element or multiple types of doping elements to substitute part of InTaO₄.

Furthermore, the cosmetic material according to the present invention is characterized in that the reduction processing is executed on InTaO₄, thereby making it enter the oxygen deficiency state. Also, with the cosmetic material according to the present invention, part of In or Ta of InTaO₄ or part of In and Ta of InTaO₄ is in the deficiency state.

### <Selection of Substitution Element>

In this embodiment, a doping element is selected from a multiplicity of elements existing on earth by performing simulations based on a first principle calculation. All analysis methods used for this analysis are based on density functional formalism. Norm-conserving pseudopotential that uses Kleinman-Bylander form (L. Kleinman and D. M. Bylander, Phys. Rev. Lett. 48 (1982) 425) for interactions between electrons and ions. A method of Troullier and Martins (N. Troullier and J. L. Martins, Phys. Rev. B. 43 (1991) 1993) is used for a pseudo-wave function and pseudopotential; and a method of Perdew and Zunger (J. P. Perdew and A. Zunger, Phys. Rev. B. 23 (1981) 5048) is used for exchange correlation energy.

Cutoff energy by plane wave energy is set so that an error between a value of a lattice constant, which corresponds to a minimum value of entire energy when calculating the entire energy of an electronic system by using the lattice constant as a function, and an experimental value becomes 5% or less. When selecting the doping element, a method of selecting the doping element through a trial and error process by repeating sample creation and evaluation experiments. However, since it can be assumed that a very large number of doping elements capable of substituting at least part of InTaO₄, and a very large number of combinations of such doping elements may exist, the efficiency of the method of repeating sample creation and evaluation experiments when selecting the doping element is very low. So, in this embodiment, the doping element is selected by a method including mainly the following two steps (a first step and a second step).

### (First Step)

Firstly, whether molecules which are formed after doping InTaO₄, from among a large number of elements that can be considered to be available as doping elements for doping InTaO₄, can exist as molecules or not (whether they are stable molecules or not) is judged based on the first principle calculation. Then, the element that can be used as the doping element is selected (screened) based on the judgment result. Whether the molecules formed after doping are stable molecules or not is judged in consideration of bond energy of the formed molecules. Specifically speaking, a permitted value of the bond energy is set in advance and then whether the bond energy of the molecules generated when at least part of InTaO₄ is substituted with a specified element becomes the permitted value or less is judged. The first principle calculation is used to derive this bond energy. Then, if the derived bond energy is equal to or less than the above-mentioned permitted value according to a simulation result based on the first principle calculation, it is determined that the relevant molecules can exist as molecules (they are stable molecule); and if the derived bond energy is equal to or more than the permitted value, it is determined that the relevant molecules cannot exist as molecules (they are unstable molecule). In other words, an element whose molecules after doping InTaO₄ become stable molecules is selected as an element capable of doping InTaO₄ (doping element) from among a large number of elements existing on earth.

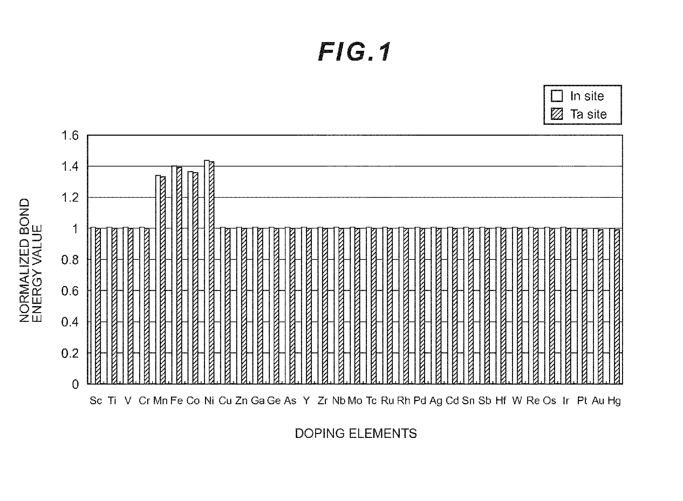

Fig. 1 illustrates the relationship between doping elements derived based on the first principle calculation and their bond energy. The horizontal axis of a graph illustrated in Fig. 1 represents the elements for doping InTaO₄ and the vertical axis represents a value obtained by normalizing a bond energy value of molecules generated after doping InTaO₄ with the element indicated on the horizontal axis, by using a bond energy value of InTaO₄ which is not doped. Incidentally, These bond energy values are derived based on the first principle calculation. Moreover, data indicated as "In site" in Fig. 1 are simulation results when In atoms of InTaO₄ were substituted with the doping element; and data indicated as "Ta site" are simulation results when Ta atoms of InTaO₄ were substituted with the doping element.

Fig. 1 shows that when any element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg is used to dope InTaO₄, its bond energy is equivalent to or higher than that of InTaO₄ which is a starting material (InTaO₄ which is not doped). Fig. 1 also shows that there is no large difference in the bond energy when the In atoms of InTaO₄ were substituted with the doping element and when the Ta atoms were substituted with the doping element. Accordingly, even when InTaO₄ is doped with each of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg, the molecules formed after doping become stable molecules and these elements are the doping elements capable of doping InTaO₄.

### (Second Step)

Next, regarding each of the doping elements which are capable of doping InTaO₄ and selected in the first step, whether molecules formed after doping InTaO₄ with the relevant doping element can be effective as a cosmetic material is judged based on the first principle calculation. Then, the doping element which can be used as the cosmetic material is selected based on that judgment result. Specifically speaking, when a certain doping element from among the plurality of doping elements selected in the first step is used to dope InTaO₄, whether molecules formed after that doping absorb the ultraviolet light or not is selected in consideration of a band gap. The first principle calculation is also used for this selection. Then, the cosmetic material capable of absorbing the ultraviolet light is selected according to the simulation result based on the first principle calculation.

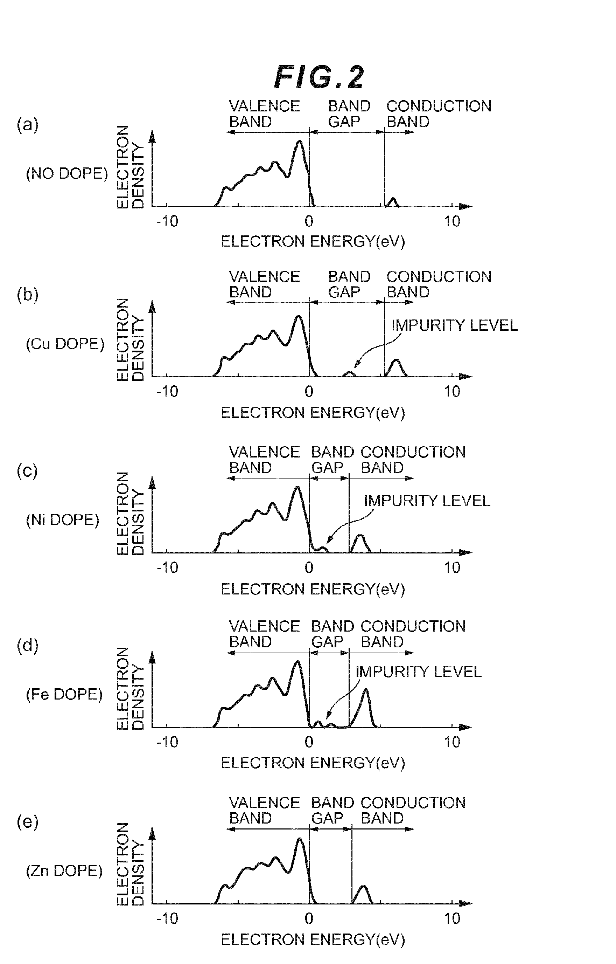

Fig. 2 to Fig. 5 illustrate the relationship between electron energy and electron density when part of InTaO₄ is substituted with the doping element. With graphs in Fig. 2(a) to Fig. 2(e), Fig. 3(a) and Fig. 3(b), Fig. 4(a) and Fig. 4(b), and Fig. 5(a) and Fig. 5(b), the horizontal axis represents the electron energy and the vertical axis represents the electron density.

Fig. 2(a) indicates data about InTaO₄ which was not doped with the doping element; and Fig. 2(b) indicates data when part of InTaO₄ was substituted with Cu. Specifically speaking, it should be noted that Fig. 2(b) indicates data when 6.25% of In of InTaO₄ was substituted with Cu. Similarly, Fig. 2(c), Fig. 2(d), and Fig. 2(e) indicate data when part of InTaO₄ was substituted with Ni, Fe, Zn, respectively. Also, Fig. 2(c), Fig. 2(d), and Fig. 2(e) indicate data when 6.25% of In of InTaO₄ was substituted with Ni, Fe, Zn, respectively.

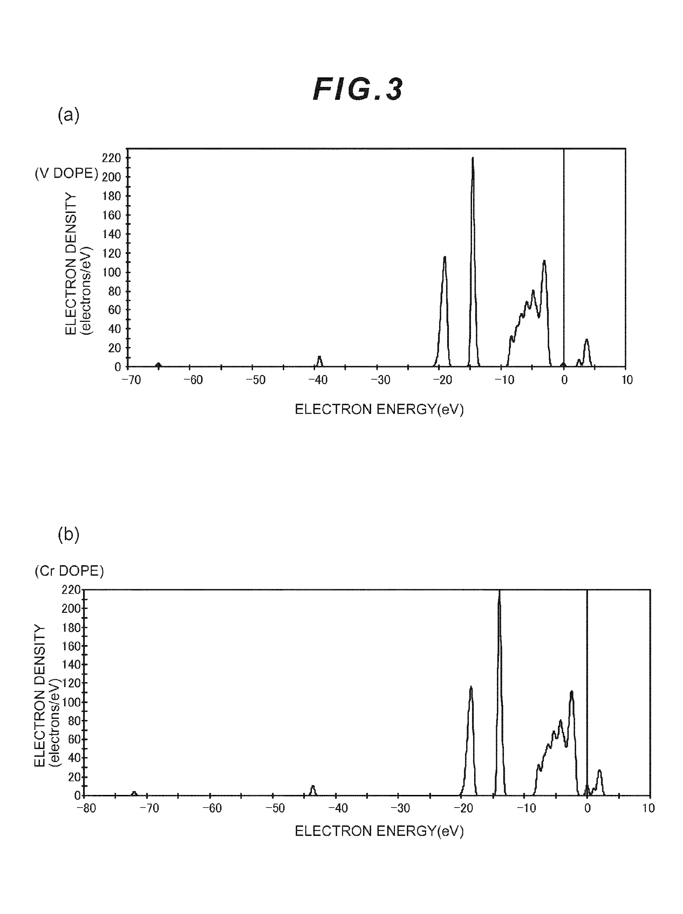

Moreover, Fig. 3(a) and Fig. 3(b) indicate data when part of InTaO₄ was substituted with V and Cr, respectively. Specifically speaking, it should be noted that Fig. 3(a) and Fig. 3(b) indicate data when 6.25% of In of InTaO₄ was substituted with V and Cr, respectively.

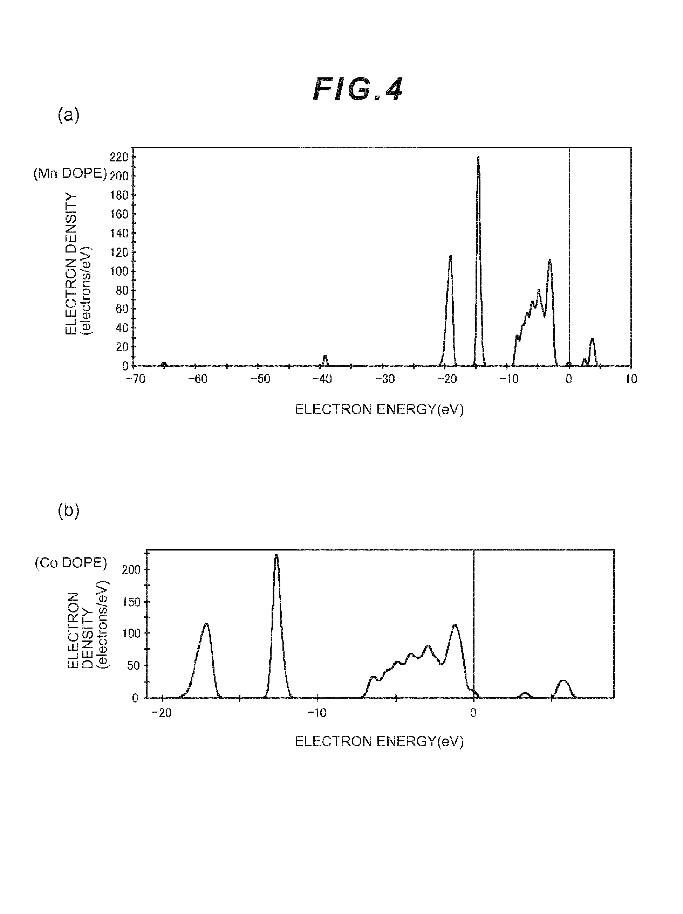

Furthermore, Fig. 4(a) and Fig. 4(b) indicate data when part of InTaO₄ was substituted with Mn and Co, respectively. Specifically speaking, it should be noted that Fig. 4(a) and Fig. 4(b) indicate data when 6.25% of In of InTaO₄ was substituted with Mn and Co, respectively.

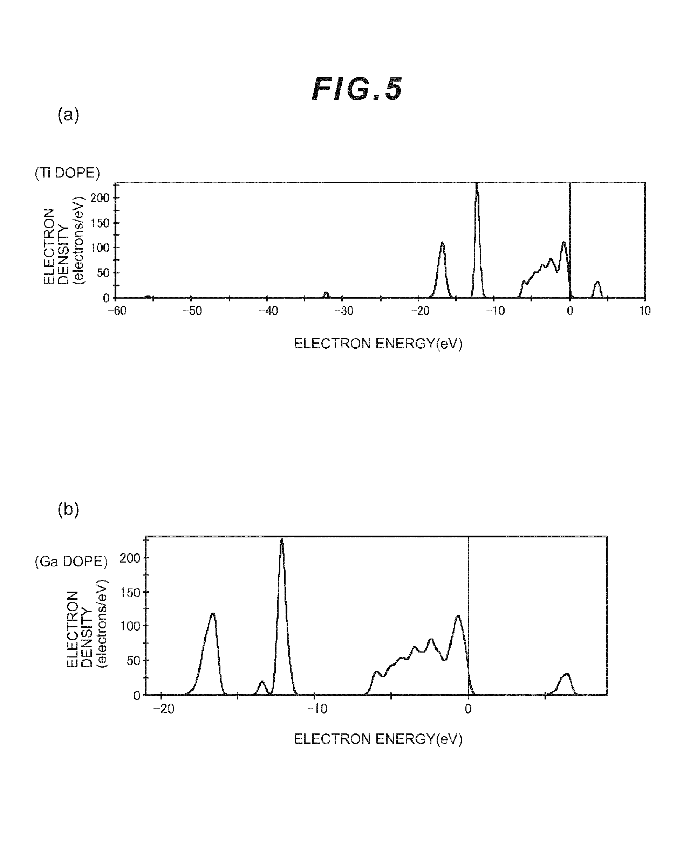

Furthermore, Fig. 5(a) and Fig. 5(b) indicate data when part of InTaO₄ was substituted with Ti and Ga, respectively. Specifically speaking, it should be noted that Fig. 5(a) and Fig. 5(b) indicate data when 6.25% of In of InTaO₄ was substituted with Ti and Ga, respectively.

For example, Fig. 2(c) shows that a conduction band shifts to a lower energy side and a band gap is reduced by doping InTaO₄ with Ni. Moreover, Fig. 2(c) also shows that the electron density of the conduction band has increased. Furthermore, Fig. 2(c) shows that a new electron level (impurity level) is formed near a valence band.

On the other hand, referring to Fig. 2(b) when the doping element is Cu, a new electron level (impurity level) is formed in isolation in the middle of the band gap. Moreover, the positions of the valence band and the conduction band are the same as those in the state of no doping with the doping element (see Fig. 2(a)) and the band gap has not changed considerably. Furthermore, since the state density of the impurity level is small, it can be assumed that light absorption in the long wavelength area between the valence band and conduction band and the impurity level is very small. Furthermore, there is a high possibility that electrons/holes excited by the light recombine at the impurity level and become obstructive factors for absorption of, for example, the ultraviolet light. In other words, the existence of the impurity level can be considered to be not suited for absorption of the ultraviolet light.

Incidentally, as depicted in Fig. 3(a) and Fig. 3(b), and Fig. 4(a) and Fig. 4(b), results similar to those of the case where Cu was used were obtained when, for example, V, Cr, Mn, or Co was used as the doping element. In other words, it can be assumed that when, for example, V, Cr, Mn, or Co is used as the doping element, molecules after doping become stable molecules, but they are not suited for absorption of the ultraviolet light.

Referring to Fig. 2(c) and Fig. 2(d) when the doping element is Ni or Fe, the position of the conduction band shifts to the lower energy side and an impurity level is formed near the valence band. The formation of the impurity level causes the band gap to reduce much; however, since the electron density of the impurity level is small, it can be assumed the light absorption by this material is small.

Referring to Fig. 2(e) when the doping element is Zn, the conduction band shifts to the lower energy side, but a new impurity level is not formed. As a result of doping with the doping element, the band gap reduces and no impurity level is formed, so that a cosmetic material capable of realizing efficient light absorption can be obtained. Moreover, as depicted in Fig. 5(a) and Fig. 5(b) when, for example, Ti or Ga was used as the doping element, results similar to those of the Zn case were obtained.

As a result, it is found that, for example, the size of the band gap, which affects light absorption property, and the electron density change considerably depending on the type of the doping element used to substitute part of InTaO₄. Then, it is found that each of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg can be used as the doping element in order to absorb the ultraviolet light; and particularly, it is desirable to use, for example, Ti, Zn, or Ga as the doping element in order to produce a cosmetic material that absorbs the ultraviolet light.

Experiments were conducted with respect to Zn, Fe, and Ni from among the plurality of doping elements selected based on the first principle calculation including the first step and the second step as described earlier. Specifically speaking, experiment objects were prepared by doping InTaO₄ with Zn, Fe, and Ni, respectively and the experiments described below were conducted. Furthermore, the same experiment was also conducted with respect to InTaO₄ which was not doped as a comparison.

A cosmetic material according to this embodiment can be synthesized by a normal solid reaction method, that is, by mixing respective metal components, which are raw materials, at target composition rates and then burning the obtained mixture in air under normal atmospheric pressure. Moreover, various methods such as various sol-gel methods and complex polymerization methods using metal alkoxides and metal salts as raw materials can be used. In this case, the solid reaction method was used to create samples of the cosmetic material. The amount of the doping element was set so that 10% substitution would be performed relative to In or Ta; and the mixture was burnt at 1150 degrees Celsius for 48 hours.

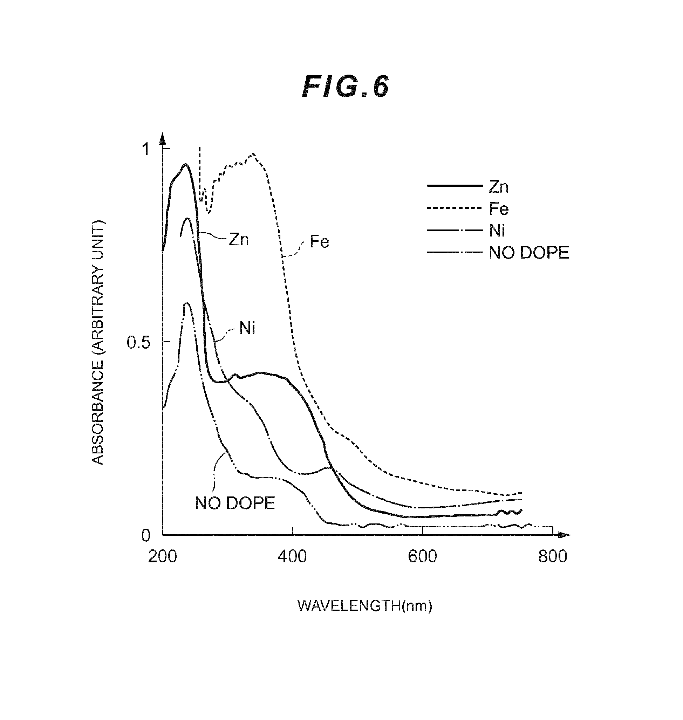

Fig. 6 illustrates absorption spectra of the created cosmetic materials (samples) by the diffuse reflectance spectroscopy method. Fig. 6 shows that when Fe is used as the doping element, the cosmetic material exhibits large absorbance in the ultraviolet light area. Incidentally, regarding the shape of the cosmetic material according to the present invention, it is desirable that it is composed of fine particles and has a large surface area in order to effectively absorb light. An oxide prepared by the solid reaction method has large particles and a small surface area; however, the particle size can be reduced by grinding the particles with, for example, a ball mill. Moreover, the fine particles can be molded into a shape and used.

By the way, the aforementioned embodiment deals with visible light of comparatively low energy by reducing the band gap as a result of the substitution of part of InTaO₄ with the doping element; however, the band gap can be narrowed by executing reduction processing on InTaO₄ and thereby making it enter an oxygen deficiency state. An example of this reduction processing will be shown below.

### <Reduction Processing>

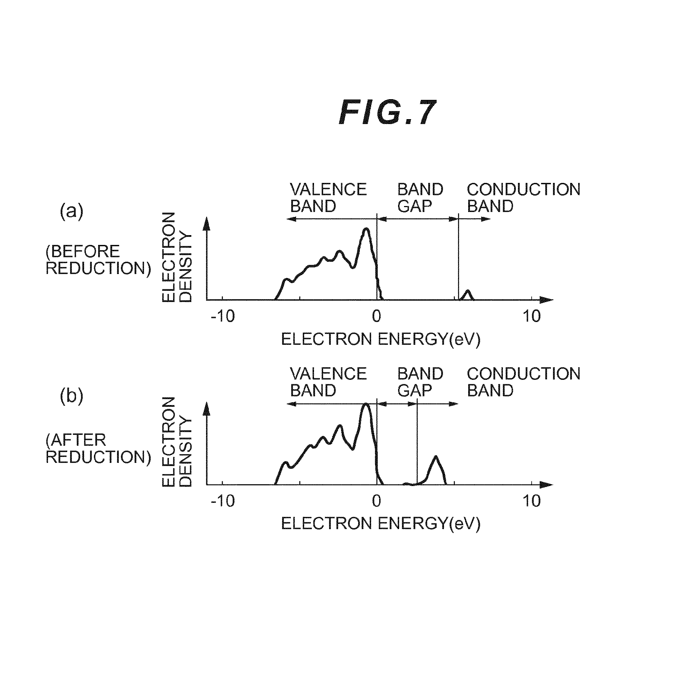

For example, processing for heating the material in hydrogen atmosphere can be listed as the reduction processing. Fig. 7(a) illustrates the relationship between the electron energy and the electron density of InTaO₄ (no dope); and Fig. 7(b) illustrates the relationship between the electron energy and the electron density when the reduction processing was executed on InTaO₄, thereby making it enter the oxygen deficiency state. Incidentally, these pieces of data are also based on the first principle calculation. Fig. 7(a) and Fig. 7(b) show that the band gap is reduced by executing the reduction processing on InTaO₄.

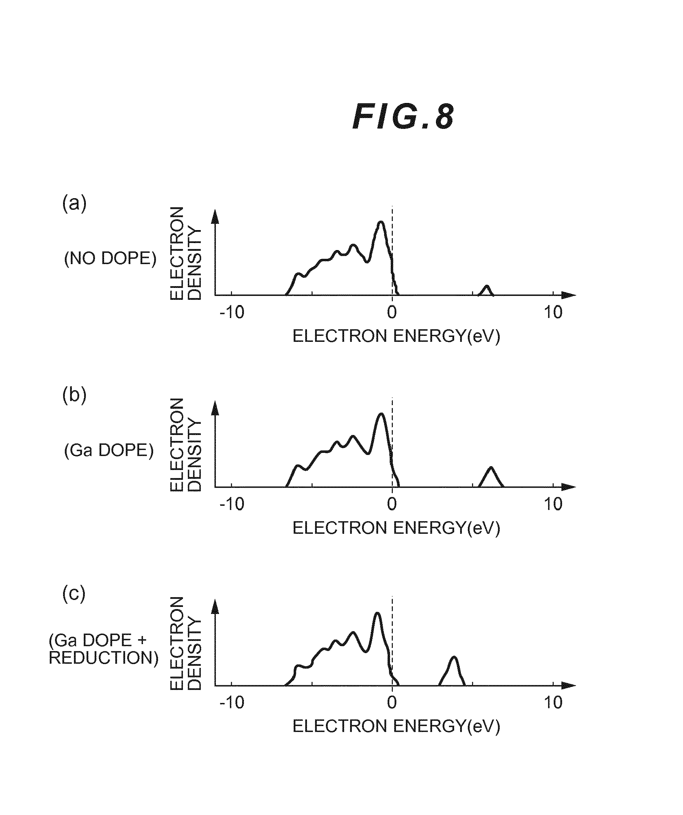

Furthermore, the material obtained after substitution with the doping element can be made to enter an oxygen deficiency state by substituting part of InTaO₄ with the doping element and then executing the reduction processing. Fig. 8(a) illustrates the relationship between the electron energy and the electron density of InTaO₄ (no dope); Fig. 8(b) illustrates the electron energy and the electron density when part of InTaO₄ is substituted with Ga as the doping element; and Fig. 8(c) illustrates the electron energy and the electron density when part of InTaO₄ is substituted with Ga as the doping element and the reduction processing is further executed on the obtained material, thereby making it enter the oxygen deficiency state. Fig. 8(b) shows that the band gap is reduced as a result of doping with Ga. Moreover, Fig. 8(c) shows that the band gap is further reduced as a result of further execution of the reduction processing. Accordingly, it has been found that the material obtained by substituting part of InTaO₄ with Ga as the doping element and the material obtained by substituting part of InTaO₄ with the doping element and then executing the reduction processing can absorb the ultraviolet light well.

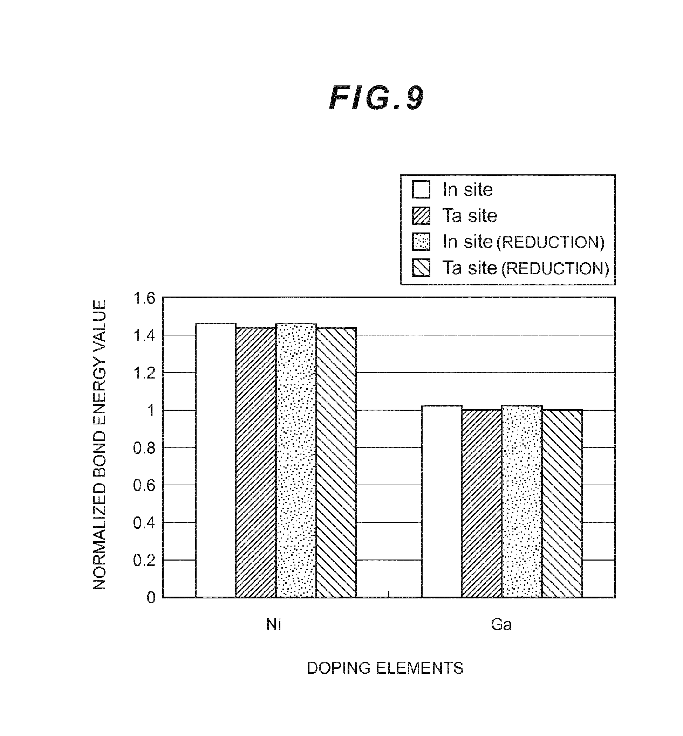

Incidentally, even when part of InTaO₄ is substituted with the doping element such as Ga and then the reduction processing is executed on the obtained material, the generated molecules become stable molecules. Fig. 9 illustrates, in an enlarged manner, simulation results obtained when Ni and Ga are used as representative examples of the doping elements from among the elements indicated on the horizontal axis of the graph depicted in Fig. 1. Referring to Fig. 9, the data indicated as "In site (reduction)" are the simulation results obtained when In atoms of InTaO₄ were substituted with the respective doping elements and then the reduction processing was executed on the obtained materials (processing for making the obtained materials enter the oxygen deficiency state); and the data indicated as "Ta site (reduction)" are the simulation results obtained when Ta atoms of InTaO₄ were substituted with the respective doping elements and then the reduction processing was executed on the obtained materials.

Fig. 9 shows that even when part of InTaO₄ is substituted with the doping element and then the reduction processing is executed on the obtained material, the resultant material has bond energy equivalent to or higher than that of InTaO₄ which is a starting material (InTaO₄ which is not substituted with the doping element). Furthermore, Fig. 9 also shows that there is no great difference in the bond energy between the case where In atoms of InTaO₄ are substituted with the doping element and the case where Ta atoms are substituted with the doping element. Furthermore, Fig. 9 shows that there is no great difference in the bond energy between before the reduction processing and after the reduction processing. As a result, it has been found that even when part of InTaO₄ is substituted with the doping element and then the reduction processing is executed on the obtained material, molecules formed after the reduction processing become stable molecules.

### <Blending Examples of Milky Lotion>

Incidentally, a blending example of a milky lotion which is an example of the cosmetic material according to the present invention is indicated as follows:
cetanol 1.0 wt%
squalene 2.0 wt%
jojoba oil 4.0 wt%
octyl paramethoxy cinnamate 4.0 wt%
polyethoxylated (10) hydrogenated castor oil 1.0 wt%
sorbitan monostearate 1.0 wt%
indium tantalum oxide powder (product of the present invention) 5.0 wt%
butylparaben 0.1 wt%
methylparaben 0.1 wt%
ethanol 3.0 wt%
glycerin 4.0 wt%
perfume material 0.05 wt%
purified water balance

### <Blending Example of Cream>

Incidentally, a blending example of a cream which is an example of the cosmetic material according to the present invention is indicated as follows:
cetanol 1.0 wt%
stearic acid 2.0 wt%
cholesterol 1.0 wt%
squalene 5.0 wt%
jojoba oil 4.0 wt%
octyl paramethoxy cinnamate 4.0 wt%
polyethoxylated (40) hydrogenated castor oil 1.0 wt%
sorbitan monostearate 2.0 wt%
indium tantalum oxide powder (product of the present invention) 7.0 wt%
butylparaben 0.1 wt%
methylparaben 0.1 wt%
ethanol 3.0 wt%
glycerin 10.0 wt%
cow placenta extract 1.0 wt%
perfume material 0.05 wt%
purified water balance

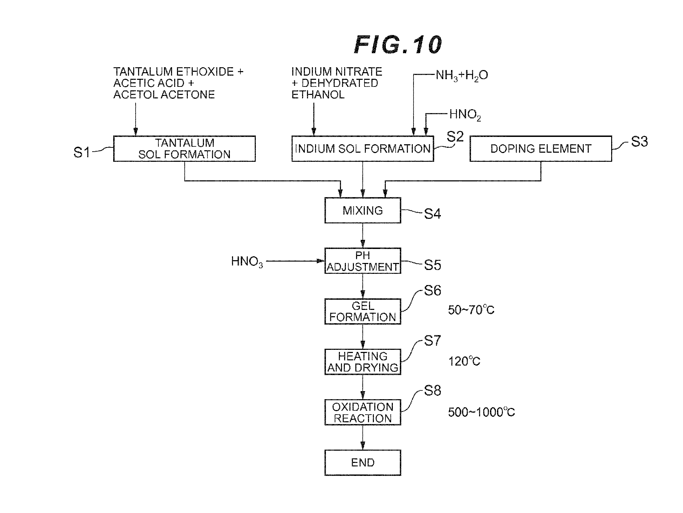

Incidentally, the cosmetic material according to this embodiment can be also produced by various sol-gel methods as mentioned earlier. Examples of the sol-gel methods can include a method including tantalum sol formation step S1, indium sol formation step S2, doping element dope step S3, mixed liquid formation step S4, PH adjustment step S5, gel formation step S6, heating and drying step S7, and oxidation reaction step S8 as shown in Fig. 10.

In the tantalum sol formation step S1, tantalum sol containing fine particles of tantalum in a colloidal state is synthesized by a chemical reaction. In this tantalum sol synthesis step S1, for example, tantalum ethoxide (Ta(OC₂H₅)₅), tantalum methoxide (Ta(OCH₃)₅), or tantalum alkoxide (Ta(OCₙH₂ₙ₊₁)₅, n=1 to 6) is used as a tantalum particle source. Moreover, in this tantalum sol formation step S1, 0 to 10 mol of acetyl acetone and 0 to 10 mol of acetic acid are mixed with 1 mol of tantalum. More specifically, firstly tantalum oxide sol (0.005 mol of tantalum ethoxide + 10 ml of acetic acid + 10 ml of acetyl acetone) is prepared by stirring it with a stirrer overnight.

Next, in the indium sol formation step S2, indium sol containing fine particles of indium in a colloidal state is synthesized by a chemical reaction. In this indium sol formation step S2, indium nitrate (In(NO₃)₃•nH₂O, n=0 to 6) or indium alkoxide (In(OCₙH₂ₙ₊₁)₃, n=1 to 6) is used as an indium particle source. Moreover, in this indium sol formation step S2, 0.1 to 10 mol of anhydrous ethanol, 0.1 to 10 mol of nitric acid, and 0.1 to 10 mol of ammonium are mixed with 1 mol of indium. More specifically, a mixture of 0.005 mol of indium nitrate + 20 ml of anhydrous ethanol is prepared; and then 1.5 ml of 25% NH₃-H₂O (approximately 0.01 mol) is slowly added to the above-obtained mixture while being stirred; and a HNO₃ solution (approximately 0.2 ml) is added to the above-obtained mixture, thereby synthesizing transparent sol.

Next, in the doping element dope step S3, the sol obtained above is substituted with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg.

Then, in the mixed liquid formation step S4, the tantalum sol, the indium sol, and the doping element are mixed, thereby forming a mixed liquid.

Subsequently, in the PH adjustment step S5, nitric acid is added as appropriate to the mixed liquid obtained in the mixed liquid formation step S4, thereby adjusting PH of the mixed liquid to become 6 to 7.

Next, in the gel formation step S6, the mixed liquid obtained in the PH adjustment step S5 is stirred with a stirrer overnight while maintaining the temperature of the mixed liquid at 50 to 70 degrees Celsius, thereby allowing the mixed liquid to gradually evaporate and enter a gel state.

Then, in the heating and drying step S7, the precursor gel obtained in the gel formation step S6 is further dried overnight at 120 degrees Celsius.

Subsequently, in the oxidation reaction step S8, an oxidation reaction of the gel dried in the heating and drying step S7 is performed at a temperature within the range from 500 to 1000 degrees Celsius, thereby forming solid InTaO₄. As a result, the cosmetic material is obtained.

Accordingly, a specific surface area of particles can be increased by atomizing the particles by the sol-gel method by using the tantalum sol, which contains the tantalum particles in the colloidal state, the indium sol, which contains the fine particles of indium in the colloidal state, and the doping element; and, therefore, the cosmetic material of nano-order grain size can be produced (synthesized). The cosmetic material obtained by using the sol-gel method can also absorb the ultraviolet light well. Moreover, the grain size of this cosmetic material is constant; and when this cosmetic material is applied to (or is fitted tightly to) the bare skin, it also has the effects of making the skin look fine-textured, transparent, natural, and beautiful.

Incidentally, the cosmetic material according to the present invention can produce color tones applicable to all races by selecting the doping element and its substitution rate. For example, color tones ranging from ochre to brownish tones can be produced by using Fe as the doping element and substituting 0.1 to 3% of at least part of InTaO₄ with Fe. Furthermore, a color tone close to black can be produced by increasing the substitution rate up to approximately 10%.

## Claims

1. A cosmetic material with at least part of InTaO₄ substituted with at least one element of Sc, Ti, V, Cr, Mn, Co, Cu, Ga, Ge, As, Y, Zr, Nb, Mo, Tc, Ru, Rh, Pd, Ag, Cd, Sn, Sb, Hf, W, Re, Os, Ir, Pt, Au, and Hg.

2. The cosmetic material according to claim 1, wherein at least part of In, Ta, or a combination thereof of InTaO₄ is substituted with the element.

3. The cosmetic material according to claim 1, wherein part of In and Ta of InTaO₄ is substituted with the element, respectively.

4. A cosmetic material with a material generated by substituting at least part of InTaO₄ with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg, being in an oxygen deficiency state as a result of reduction processing.

5. The cosmetic material according to claim 4, wherein at least part of In, Ta, or a combination thereof of InTaO₄ is substituted with the element.

6. The cosmetic material according to claim 4, wherein at least part of In and Ta of InTaO₄ is substituted with the element, respectively.

7. A cosmetic material with InTaO₄ in an oxygen deficiency state as a result of reduction processing.

8. A cosmetic material with part of In or Ta of InTaO₄ or part of In and Ta of InTaO₄ being in a deficiency state.

9. A cosmetic material production method comprising a substitution step of substituting at least part of InTaO₄ with at least one element of Sc, Ti, V, Cr, Mn, Co, Cu, Ga, Ge, As, Y, Zr, Nb, Mo, Tc, Ru, Rh, Pd, Ag, Cd, Sn, Sb, Hf, W, Re, Os, Ir, Pt, Au, and Hg.

10. The cosmetic material production method according to claim 9, wherein in the substitution step, at least part of In, Ta, or a combination thereof of InTaO₄ is substituted with the element.

11. The cosmetic material production method according to claim 9, wherein in the substitution step, part of In and Ta of InTaO₄ is substituted with the element, respectively.

12. A cosmetic material production method comprising:
a substitution step of substituting at least part of InTaO₄ with at least one element of Sc, Ti, V, Cr, Mn, Fe, Co, Ni, Cu, Zn, Ga, Ge, As, Y, Zr, Nb, Mo, Tc, Ru, Rh, Pd, Ag, Cd, Sn, Sb, Hf, W, Re, Os, Ir, Pt, Au, and Hg; and
an oxygen deficiency step of executing reduction processing on a material obtained above after the substitution step and thereby making it enter an oxygen deficiency state.

13. The cosmetic material production method according to claim 12, wherein in the substitution step, at least part of In, Ta, or a combination thereof of InTaO₄ is substituted with the element.

14. The cosmetic material production method according to claim 12, wherein in the substitution step, part of In and Ta of InTaO₄ is substituted with the element, respectively.

15. A cosmetic material production method comprising an oxygen deficiency step of executing reduction processing on InTaO₄ and making it enter an oxygen deficiency state.
